# EUROPEAN PATENT APPLICATION

(11) **EP 4 155 402 A1**
(43) Date of publication of application: **29.03.2023**
(21) Application number: 21198390.3
(22) Date of filing: 22.09.2021
(51) Int. Cl.: C12N 15/113, A61K 31/7105, A61K 39/00, A61P 25/00, A61P 25/08

(54) **MODULATION OF MICRORNA-335 FOR THE TREATMENT OF SODIUM CHANNELOPATHIES**

(71) Applicant: Royal College of Surgeons in Ireland, Dublin 2 (IE)
(72) Inventor: Henshall, David, Dublin 2 (IE); Morris, Gareth, Dublin 2 (IE); Heiland, Mona, Dublin 2 (IE)
(74) Representative: Purdylucey Intellectual Property

(57) **Abstract**

A modulator of miR-335 for use in the treatment of a sodium channelopathy in a subject is provided. Typically, the modulator of miR-335 is to be delivered to the brain of the subject. Preferably, the sodium channelopathy is a neurological condition.

## Description

### Field of the Invention

The current invention relates to a therapeutic strategy to treat diseases or conditions associated with dysregulation in genes encoding voltage-gated sodium channels, such as epilepsy and autism. In particular, the invention relates to miR-335 modulation in the brain of a subject to treat such diseases or conditions.

### Background of the Invention

Voltage-gated sodium channels (NaV) play an essential role in the generation and propagation of action potential, and malfunction caused by mutations can induce irregular neuronal activity. NaVs are composed by an α subunit organized in four homologous ligated domains (DI-DIV), each domain composed by six transmembrane segments (S1-S6), and one or more β subunits associated by non-covalent interactions or disulfide bond. There are several different alpha subtypes of NaVs and mutations in these channels can cause diseases or conditions known as sodium channelopathies.

One example is epilepsy. Epilepsy is a serious, chronic neurologic disorder characterised by recurrent spontaneous seizures which affects about 50 million people worldwide. Certain types of epilepsy, among other conditions such as autism, are caused by mutations to voltage-gated sodium channel genes, such as SCN1A and SCN2A, which are involved in brain excitability. This group of diseases or conditions are collectively called sodium channelopathies.

Genetic epilepsies typically begin very early in life and are associated with severe developmental deficits and drug-resistant seizures. Genetic epilepsies caused by these mutations do not currently have adequate treatment options. Moreover, clinically available anti-seizure drugs cause wide ranging adverse effects due to global dampening of brain activity.

Current treatment options for sodium channelopathies are limited. Anti-seizure drugs remain the frontline approach, though their efficacy varies between different syndromes. Epidiolex (CBD) is emerging as a treatment for SCN1A channelopathies (Dravet syndrome), and novel approaches in pre-clinical development include TANGO (Lim, K.H., et al. Antisense oligonucleotide modulation of non-productive alternative splicing upregulates gene expression. Nat Commun 11, 3501 (2020)) and CRISPR-based approaches (Yamagata T, et al., CRISPR/dCas9-based Scn1a gene activation in inhibitory neurons ameliorates epileptic and behavioral phenotypes of Dravet syndrome model mice. Neurobiol Dis. 2020 Jul;141:104954; and Colasante G, et al., dCas9-Based Scn1a Gene Activation Restores Inhibitory Interneuron Excitability and Attenuates Seizures in Dravet Syndrome Mice. Mol Ther. 2020 Jan 8;28(1):235-253). Sodium channel blockers are effective in some SCN2A channelopathies but can also exacerbate symptoms depending on the effect of the mutation on the channel. They do not address the underlying cause of the disease. Other pre-clinical efforts to manipulate SCN2A include use of an alternative splice event (WO2020154462) or antisense oligonucleotides against SCN2A itself (EP3840733).

There is an urgent need for innovative treatment strategies which better control seizures, assist with developmental deficits, and/or have fewer or no adverse effects.

miRNAs are a family of small (~22nt), endogenously expressed non-coding RNAs which regulate mRNA translation by imperfect base-pairing interactions within the 3' untranslated region. Depending on the degree of sequence complementarity, miRNA binding, which occurs via Argonaute proteins within the RNA-induced silencing complex (RISC), results in either cleavage or a reduction in the translational efficiency of the target mRNA.

Insight into the role of miRNAs in disease development has made miRNAs attractive tools and targets for novel therapeutic approaches. However, no such treatment for sodium channelopathies is available to date.

The current invention serves to alleviate the problems of the prior art.

### Summary of the Invention

The current inventors have identified microRNA-335 (miR-335) as a naturally occurring molecule in the brain which normally acts to dampen the expression of several key genes associated with brain excitability, such as SCN1A and SCN2A. The inventors show experimental manipulation of miR-335 can alter the expression of these genes and, ultimately, modulate brain excitability.

Thus, miR-335 is a new therapeutic target for sodium channelopathies. It represents a precision approach to manipulate or restore the function of specific voltage-gated sodium channel genes.

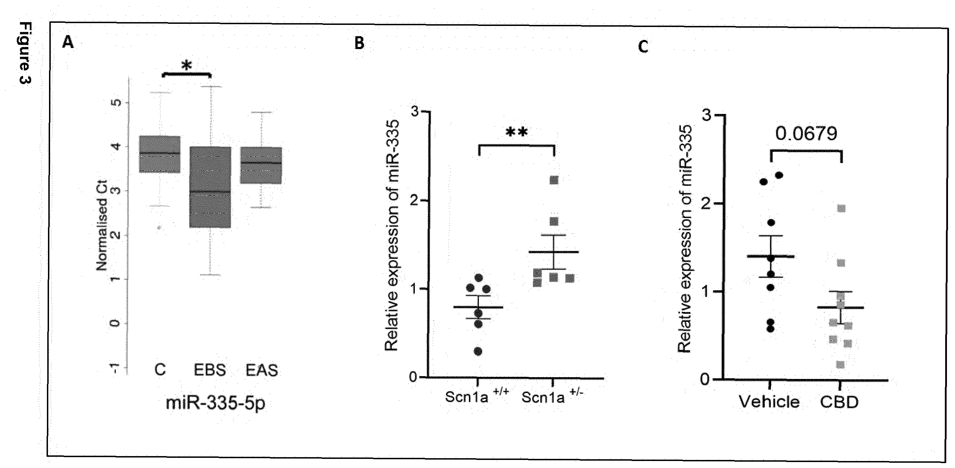

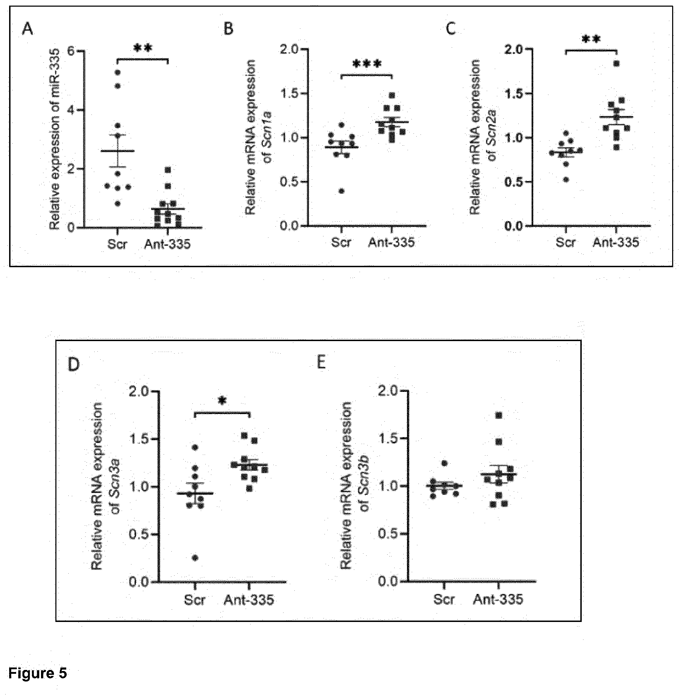

The inventors have shown that miR-335 levels are upregulated in two different types of epilepsy (Figure 3), human temporal lobe epilepsy and Dravet syndrome. The inventors have further shown that ant-335, an miR-335 antagomir, upregulates the expression of sodium channels in the mouse chain (Figure 5) with upregulation of SCN1A, SCN2A and SCN3A. AAV9-miR-335 treatment upregulated the expression of miR-335 in the mouse brain and decreased the seizure severity in a mouse model.

As miR-335 has been shown to be upregulated in epilepsy, the current method is surprising as antagonism of miR-335, to restore it to normal levels, actually exacerbates certain epilepsies, due to the precise mechanism of action.

This is the first time an interaction between miR-335 and sodium channels, such as voltage-gated sodium channels, has been shown.

An aspect of the invention provides a modulator of miR-335 (herein referred to as "modulator of the invention") for use in the treatment or prevention of a sodium channelopathy in a subject.

In an embodiment, the sodium channelopathy is a neurological sodium channelopathy. It may be one associated with the occurrence of seizures. It may be a neurodevelopmental disorder.

The sodium channel may be a voltage-gated sodium channel, or a sodium leak channel.

In an embodiment, the sodium channelopathy is associated with, or caused by, a loss of function mutation in a voltage-gated sodium channel gene. In said embodiment, the modulator is an inhibitor of miR-335.

In an embodiment, the sodium channelopathy is associated with, or caused by, a gain of function mutation in a voltage-gated sodium channel gene. In said embodiment, the modulator is one that increases miR-335.

Preferably, the sodium channelopathy is an SCN1A sodium channelopathy. Typically, said SCN1A sodium channelopathy includes but is not limited epilepsy, Dravet syndrome and familial hemiplegic migraine-3 (FHM3). The epilepsy may be genetic epilepsy. The genetic epilepsy may be developmental and epileptic encephalopathy 6B (DEE6B), familial hemiplegic mirgraine-3, a disease associated with T226M NaV.1.1 mutation, generalised epilepsy with febrile seizures plus type 2 (GEFSP2) and severe myoclonic epilepsy of infancy (SMEI).

Preferably, the SCN1A sodium channelopathy may be one associated with a loss of function mutation in SCN1A. The SCN1A sodium channelopathy may be one associated with a gain of function mutation in SCN1A.

In an embodiment, the sodium channelopathy is an SCN2A sodium channelopathy. Typically, said SCN2A sodium channelopathy is selected from but not limited to epilepsy, e.g., genetic epilepsy, and autism, such as ASD, and intellectual disability with later onset mild epilepsy or without epilepsy. The epilepsy may be selected from the group comprising developmental and epileptic encephalopathy 11 (DEE11), episodic ataxia type 9 (EA9), benign familial infantile seizures-3 (BFIS3), Ohtahara syndrome and human temporal lobe epilepsy (TLE).

Preferably, the SCN2A sodium channelopathy may be one associated with a gain of function mutation in SCN2A. Still preferred, the SCN2A sodium channelopathy may be one associated with a loss of function mutation in SCN2A.

In an embodiment, the sodium channelopathy is an SCN1A and SCN2A sodium channelopathy.

In an embodiment, the sodium channelopathy is an SCN3A sodium channelopathy. SCN3A sodium channelopathies may be epilepsy, preferably, genetic epilepsy, such as those selected from the group comprising developmental and epileptic encephalopathy-62 (DEE62), a disease associated with a p.Leu247Pro mutation in Nav1.3, developmental and epileptic encephalopathy, and familial focal epilepsy with variable foci-4 (FFEVF4).

Preferably, the SCN3A sodium channelopathy may be one associated with a gain of function mutation in SCN3A. Still preferred, the SCN3A sodium channelopathy may be one associated with a loss of function mutation in SCN3A.

In an embodiment, the sodium channelopathy is an SCN9A sodium channelopathy. SCN9A sodium channelopathies may be selected from the group comprising primary erythermalgia, congenital insensitivity to pain, complete insensitivity to pain (CIP), anosmia (loss of sense of smell, primary erythromelalgia (PEM), neuropathy hereditary sensory and autonomic type IID, paroxysmal extreme pain disorder, inherited erythromelalgia (IEM) and small fiber neuropathy.

Preferably, the SCN9A sodium channelopathy may be one associated with a gain of function mutation in SCN9A. Still preferred, the SCN9A sodium channelopathy may be one associated with a loss of function mutation in SCN9A

In an embodiment, the sodium channelopathy is an SCN4A sodium channelopathy. SCN4A encloses a sodium voltage gated channel, alpha subunit 4. SCN4A sodium channelopathies may be selected from the group comprising hyperkalemic periodic paralysis type 2, hypokalemic periodic paralysis type 2, myasthenic syndrome congenital 16, myotonia congenita (atypical) acetazolamide-responsive and paramyotonia congenita.

Preferably, the SCN4A sodium channelopathy may be one associated with a gain of function mutation in SCN4A. Still preferred, the SCN4A sodium channelopathy may be one associated with a loss of function mutation in SCN4A

An aspect of the invention provides a modulator of miR-335 ("modulator of the invention") for use in the treatment or prevention of a UBE3A associated condition (ubiquitin-protein ligase E3A). The condition may be Angelman's syndrome or Prader-Willi Syndrome.

In an embodiment, the modulator of the invention is one capable of inhibiting miR-335. It may be an agent capable of inhibiting miR-335 activity.

In an embodiment of the present invention, the agent inhibiting miR-335 is selected from the group comprising: antagomirs, antimiRs, microRNA sponges including circular RNAs, tiny seed-targeting LNA oligonucleotides, antisense oligonucleotides, short-interfering RNA, decoy oligonucleotides, aptamers, and antibodies that specifically recognize DNA:RNA heteroduplexes.

MiR-335 may be inhibited by gene therapy methods.

In an embodiment of the current invention, the modulator of the invention is one capable of increasing miR-335. It may be capable of increasing miR-335 activity. This may be by miRNA replacement therapy using miRNA-335 mimic. The modulator may be an agent such as miR-335 or an miR-335 mimic. miR-335 or said mimic may be provided or delivered as a vector comprising miR-335 or said mimic.

MiR-335 may be increased by gene therapy methods.

The modulator may be provided as a composition comprising the modulator of the invention. The composition may be a pharmaceutical composition.

As such, in a further aspect, there is provided a composition, typically a pharmaceutical composition, comprising the modulator of the invention.

A further aspect provides a miR-335 modulator or a composition comprising the miR-335 modulator of the invention for use as a medicament.

An aspect provides a composition, typically a pharmaceutical composition comprising the modulator of the invention for use the treatment of a sodium channelopathy in a subject.

An aspect provides a composition, typically a pharmaceutical composition comprising the modulator of the invention for use the treatment of a UBE3A associated condition (ubiquitin-protein ligase E3A). The condition may be Angelman's syndrome or Prader-Willi Syndrome.

The pharmaceutical composition of any aspect may further comprise a pharmaceutically acceptable excipient.

In an embodiment, the agent is delivered to the brain of the subject. This may be direct delivery or indirect delivery.

### Brief Description of the Figures

The invention will now be described with reference to the following figures in which;
**Figure** Error! No text of specified style in document.1: Intracerebroventricular injection.

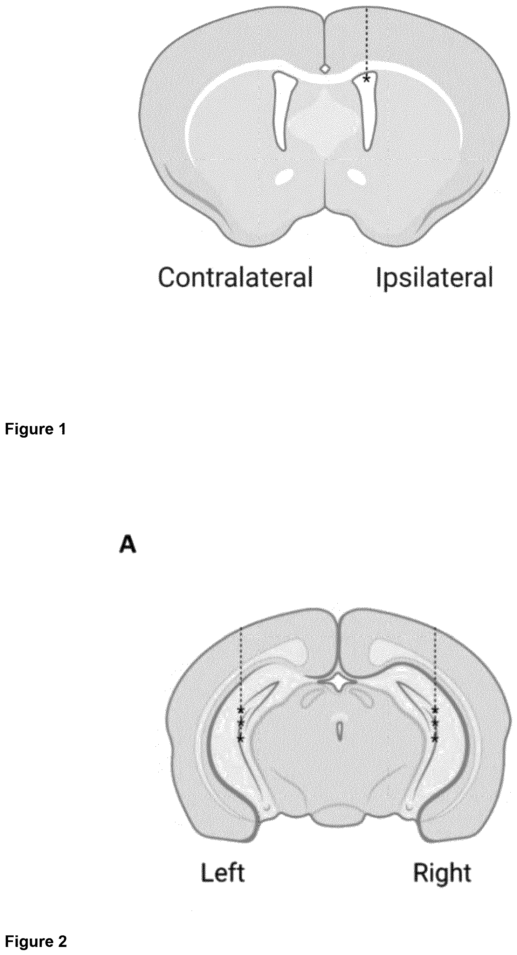

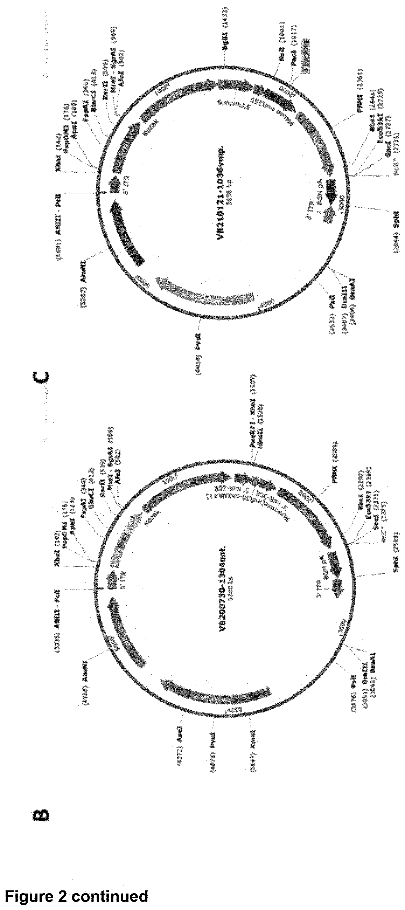

**Figure 2****:** Viral vector administration. A Coordinates for injection of viral vector. B Vector map for AAV9-miR-335. C Vector map for AAV9-Scr.
**Figure 3****:** miR-335 levels are upregulated in two different types of epilepsy. A Levels of miR-335 are upregulated in human temporal lobe epilepsy (P = 0.017). B RT-qPCR identified increased levels of miR-335 in a mouse model of Dravet syndrome (P = 0.0087, n = 6 per group). C Naïve mice treated with CBD show decreased levels of miR-335 (n = 8-9 per group). *P < 0.05, **P < 0.01 compared to control group.

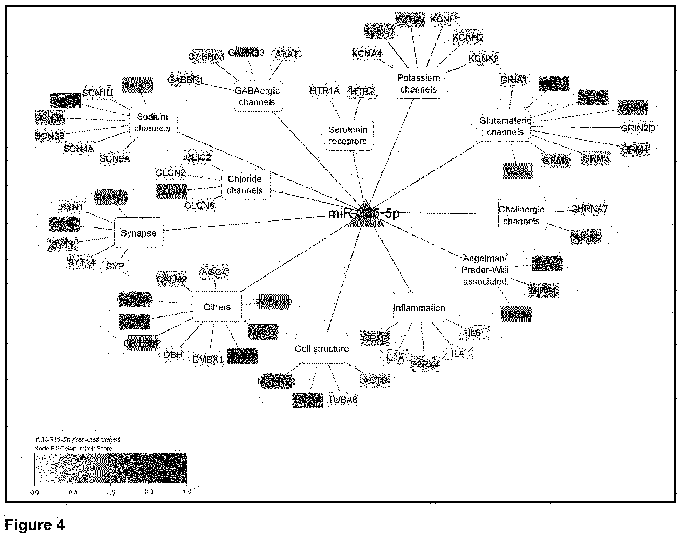

**Figure 4****:** *In silico* predictions of miR-335 target genes. MiR-335 targets many different types of channels, including sodium channels. SCN2A is amongst the highest confidence predictions (dark blue colour).
**Figure 5****:** Ant-335 upregulates the expression of different sodium channels in mouse brain. A Ant-335 reduces the expression of miR-335 (P = 0.012, n = 9-11 per group). This results in the upregulation of *Scn1a* (P = 0.0009, B), *Scn2a* (P = 0.012, C) and *Scn3a* (P = 0.0287, D), where levels of *Scn3b* (E) are unchanged (n = 9-10 per group). *P < 0.05, **P < 0.01, ***P < 0.001 compared to control group.

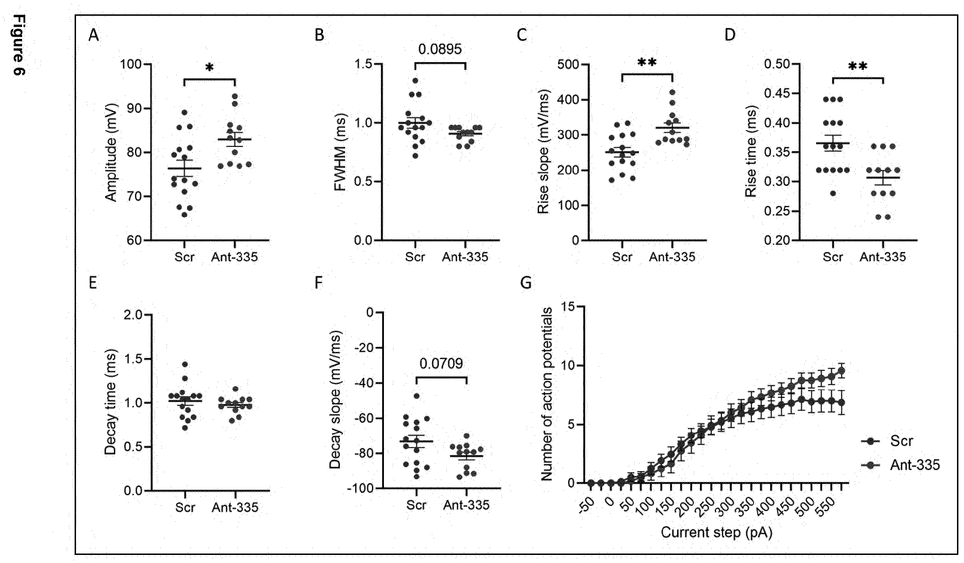

**Figure 6****:** Ant-335 increases excitability of hippocampal pyramidal neurons in line with an increase in functional voltage-gated sodium channels. **(A)** Ant-335 increased action potential amplitude (p=0.012). **(B)** Action potential width did not change significantly. **(C)** The rising slope and **(D)** rise time were significantly accelerated by ant-335 (p=0.0016 and p=0.0043 respetively), indicative of enhanced voltage-gate sodium current. **(E)** and **(F)** Action potential decay time and slope were not significantly changed, highlighting the specificity of the effect on voltage-gated sodium currents. **(G)** Ant-335 increased the maximum firing frequency of pyramidal neurons, which were able to fire above the plateau seen in control neurons (RM two-way ANOVA current x treatment p<0.0001).

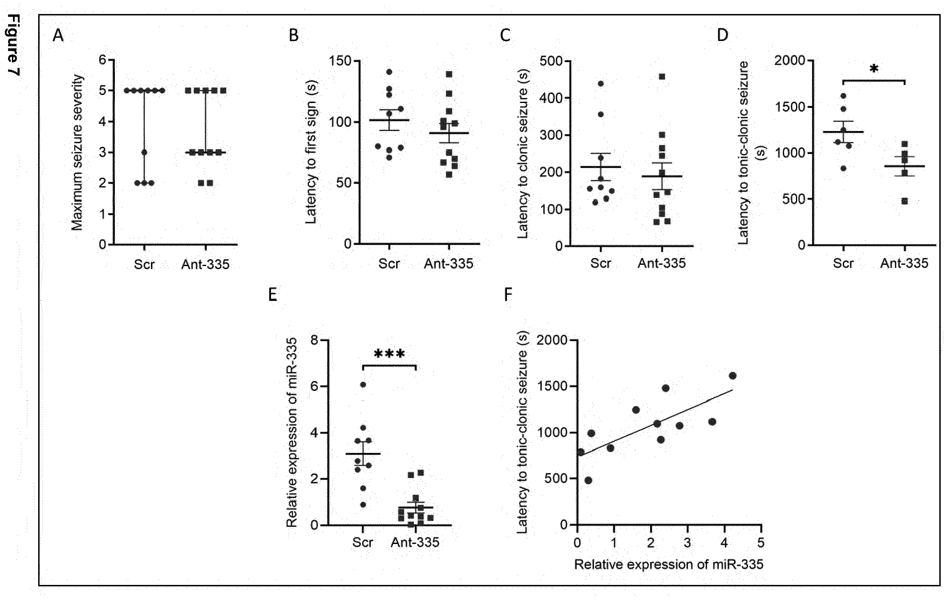

**Figure 7****:** Ant-335 treatment increases susceptibility to tonic-clonic seizures in PTZ mouse model. **A** Maximum seizure severity. **B** Latency to first sign of seizure. **C** Latency to first clonic seizure. **D** Latency to tonic-clonic seizure (P = 0.0453). **E** Ant-335 treatment decreases levels of miR-335 (P = 0.0003). **F** Correlation between the levels of miR-335 and the latency to tonic clonic seizures (R² = 0.556). N = 9-11 per group. *P < 0.05, **P < 0.01, ***P < 0.001 compared to control group.

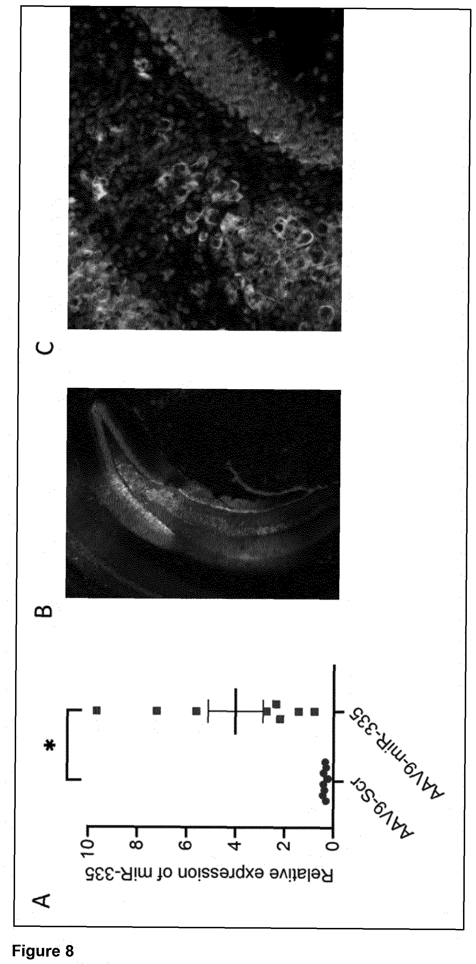

**Figure 8****:** AAV9-miR-335 upregulates the expression of miR-335 in mouse brain. **A** Levels of miR-335 are upregulated two weeks after the injection of AAV9-miR-335 (P = 0.0135, n = 4 per group, dorsal and ventral hippocampi pooled). **B** Expression of AAV9 virus (green) after intra-hippocampal injection in ventral hippocampus. **C** AAV9 is expressed in neurons (NeuN in red, DAPI in blue) in the pyramidal cells of the CA3 region. *P < 0.05 compared to control group.

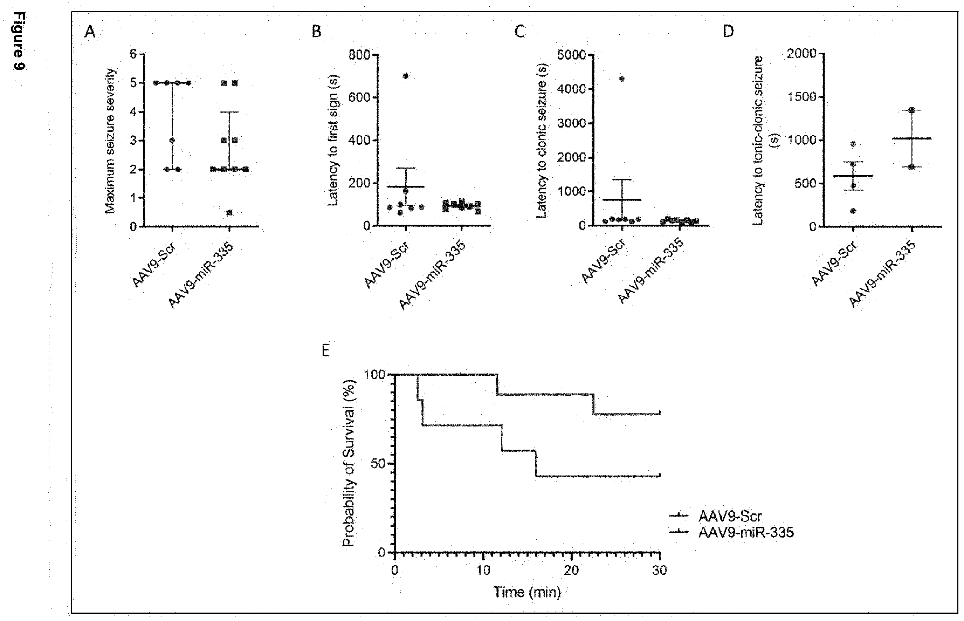

**Figure 9****:** AAV9-miR-335 seems to decrease the seizure severity in the PTZ mouse model. **A** Seizure severity. **B** Latency to first sign of a seizure. **C** Latency to first clonic seizure. **D** Latency to tonic-clonic seizure. **E** Animals treated with AAV9-miR-335 have a higher survival rate than control mice. N = 7-9 per group.

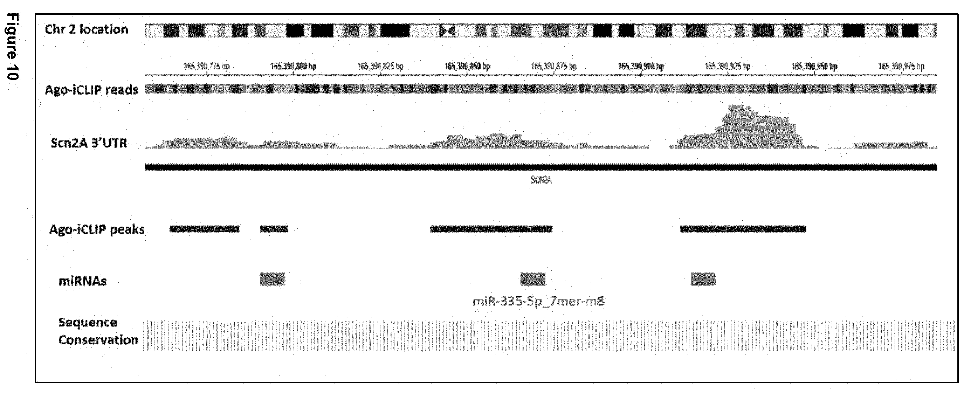

**Figure 10****:** The interaction between miR-335-5p and SCN2A is preserved in human brain. iCLIP data from human epilepsy patients identified that the SCN2A gene which is located in chromosome 2, has a target site for miR-335-5p. The orange bars represent the aggregated iCLIP reads across all the patient samples that map along the SCN2A gene.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g., a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refer to an intervention (e.g., the administration of an agent to a subject) which cures, ameliorates or lessens the symptoms of a disease or removes (or lessens the impact of) its cause(s). In this case, the term is used synonymously with the term "therapy". It can be manifested by a permanent or temporary improvement in the subject's condition. In this context it includes limiting and/or reversing disease progression.

As used herein the terms "prevention" or "preventing" refer to an intervention (e.g., the administration of an agent to a subject), which prevents or delays the onset or progression of a disease in a subject or reduces (or eradicates) its incidence within a treated population.

When used herein, the term "composition" should be understood to mean something made by the hand of man, and not including naturally occurring compositions. Compositions may be formulated in unit dosage form, i.e., in the form of discrete portions containing a unit dose, or a multiple or sub-unit of a unit dose.

When used herein, the term "pharmaceutical composition" may comprise one or more pharmaceutically acceptable diluents, excipients or carriers. Even though the peptides and compositions of the present invention can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients", 2^{nd} Edition, (1994), edited by A Wade and PJ Weller. In particular, formulations for topical delivery are described in "Topical Drug Delivery Formulations" edited by David Osborne and Antonio Aman, Taylor & Francis, the complete contents of which are incorporated herein by reference. Acceptable carriers or diluents for therapeutic use are well known in the pharmaceutical art, and are described, for example, in Remington's Pharmaceutical Sciences, Mack Publishing Co. (A. R. Gennaro edit. 1985).

As used herein, the term "effective amount or a therapeutically effective amount" as applied to the modulator of the invention or composition of the invention defines an amount that can be administered to a subject without excessive toxicity, irritation, allergic response, or other problem or complication, commensurate with a reasonable benefit/risk ratio, but one that is sufficient to provide the desired effect, e.g., modulation of miR-335 and treatment of a sodium channelopathy. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result need not be a complete cure. A therapeutic result may be a permanent or temporary improvement in the subject's condition. In this context it may also include an amount which results in a clinically significant inhibition, amelioration, decrease in severity of, or reversal of development or occurrence of seizures.

The term "symptom" is defined as an indication of disease, illness, injury, or that something is not right in the body. In this context, the symptom may be but is not limited to the occurrence of seizures or a decrease in the severity of seizures.

The term "subject" means a human or animal, more typically a mammal. In one aspect, the subject is a human.

The term "microRNA's (miRNAs)" refers to small (~22nt) non-coding RNAs (ncRNAs) that regulate gene expression at the level of translation. Each miRNA apparently regulates multiple genes and hundreds of miRNA genes are predicted to be present in mammals. Recently miRNAs have been found to be critical for development, cell proliferation and cell development, apoptosis and fat metabolism, and cell differentiation.

In the specification, the term "miR-335" should be understood to mean microRNA-335, e.g., microRNA-335-5p.

Mature human miR-335 has the following sequence (SEQUENCE ID NO. 1): ucaagagcaauaacgaaaaaugu
Hsa-miR-335 stem loop has the following sequence: 
UGUUUUGAGCGGGGGUCAAGAGCAAUAACGAAAAAUGUUUGUCAUAAACCGUUUUUC
AUUAUUGCUCCUGACCUCCUCUCAUUUGCUAUAUUCA (SEQUENCE ID NO. 2)
Mmu-miR-335 stem loop has the following sequence:

UCUUUUGGGCGGGGGUCAAGAGCAAUAACGAAAAAUGUUUGUUUUUCGUAAACCGU
UUUUCAUUAUUGCUCCUGACCCCCUCUCAUGGGUUAUAGCCA (SEQUENCE ID NO. 3)

The term "human miR-335" or hsa-microRNA-335-5p should also be taken to include any human miR-335 paralogues. It will be appreciated that sequence variations of miR-335 may occur between species.
In the specification, the term "sodium channelopathy" refers to a disease or condition caused by or associated with a mutation to a sodium channel gene or genes. The sodium channel may be a voltage-gated sodium channel or a sodium leak channel (non-selective). Preferably, the sodium channel is a voltage-gated sodium channel. The gene(s) may be one or more of SCN1A, SCN2A, SCN3A, SCN9A, SCN8A and SCN4A, preferably SCN1A, SCN2A and/or SCN3A. When the channel is a sodium leak channel, the gene may be NALCN. For example, a disease or condition associated or caused by a mutation in SCN1A would be called an SCN1A sodium channelopathy. The mutation may be a loss of function mutation, or a gain of function mutation. Typically, the sodium channelopathy is a neurological condition, such as a neurodevelopmental disorder. It may or may not include developmental impairment and/or intellectual disability. Means to determine a mutation in one or more of the genes noted above are known to the skilled person. Examples are disclosed in for example Meisler, M.H., et al., Sodium channelopathies in neurodevelopmental disorders. Nat Rev Neurosci 22, 152-166 (2021), Luis Felipe Santos Menezes et al., Epilepsy-related voltage-gated sodium channelopathies: A Review., Front. Pharmacol. 18 August 2020, and Miriam H. Meisler, et al., Sodium channel mutations in epilepsy and other neurological disorders, J Clin Invest. 2005; 115(8): 2010-2017.

The term "gain of function mutation" refers to a type of mutation in which the altered gene product has a new molecular function or enhanced molecular function or a new pattern of gene expression.

The term "loss of function mutation" refers to a type of mutation which result in the gene product having less or no function, e.g., being partially or wholly inactivated.

In the specification, the term "modulator of miR-335" is an agent or process that modulates or changes miR-335 in a subject. It may inhibit the activity or enhance or increase the activity. It may act by changing, i.e., increasing or decreasing, the expression level or the amount.

In the specification, the term "inhibition of miR-335", e.g., activity, should be understood to mean preventing miR-335 carrying out its function(s). Generally, inhibiting miR-335 should be understood to include direct inhibition in which a molecule binds to miR-335 and directly inhibits its activity (for example, a low molecular weight inhibitor or a binding partner such as an antibody or antibody fragment) and indirect inhibition in which, for example, expression of the miRNA molecule is modulated by suitable means, including for example use of repressors or small interfering RNA molecules. Inhibition of miR-335 function should be understood to encompass administering any molecule which directly or indirectly inhibits the mature form of miR-335 (SEQ ID NO:1), or part thereof, or the miR-335 stem loop (SEQ ID NO. 2) or part thereof, or any human miR-335 paralogues. It also includes agents and means for degradation of miR-335 to reduce the amount in the subject. It will be appreciated that the means of inhibition may be by gene therapy. It will also be understood to include an agent that is a target site blocker (TSB). It includes the agents capable of inhibiting miR-335 as disclosed herein.

In the specification, the term "increase miR-335", e.g., activity" should be understood to mean enhancing or encouraging miR-335 function(s). Generally, increasing miR-335 activity should be understood to include direct increase, such as an miRNA mimic, or indirect increase in which, for example, expression of the miRNA molecule is modulated by suitable means. It will be appreciated that the means of increasing miR-335 may be by gene therapy.

The term should also include administering molecules which interfere with miR-335 function by enhancing expression of its target mRNA transcripts.

In the specification, the term "agent capable of inhibiting miR-335" should be understood to mean any agent which prevents miR-335 carrying out its function(s), e.g., one that may inhibit, ideally specifically inhibit, the expression or activity or level of miR-335. Suitable agents include antagomirs, antisense molecules, small hairpin RNA molecules, small interfering RNA molecules, microRNA sponges, tiny seed-targeting locked nucleic acid (LNA) oligonucleotides, decoy oligonucleotides, aptamers, ribozymes, or antibodies that specifically recognize DNA:RNA heteroduplexes. Small hairpin RNA (shRNA) molecules are short RNA molecules having a small hairpin loop in their tertiary structure that may be employed to silence genes. The design of shRNA molecules capable of inhibiting miR-335 will be apparent to those skilled in the field of shRNA molecule design. As an alternative, the level of miR-335 expression can be modulated using antisense or ribozyme approaches to inhibit or prevent miR-335 activity, or triple helix approaches to inhibit transcription of miR-335. Agents also include target site blockers (TSB), gene therapy agents and agents capable of degrading miR-335.

In the specification, the term "antagomir" should be understood to mean a novel class of chemically engineered oligonucleotides. Antagomirs are used to silence endogenous microRNA. An antagomir is a small synthetic oligonucleotide that is complementary to the specific miRNA target with either mispairing at the cleavage site of Arganoute 2 (Ago2) or some sort of base modification to inhibit Ago2 cleavage. Usually, antagomirs have some sort of modification, such as 2' methoxy groups, 3'-cholesterol groups, phosphorothioates, to make it more resistant to degradation. It is believed that antagomirs inhibit by irreversibly binding the miRNA. An example of antagomirs suitable for use in inhibiting the activity of miR-335 are molecules comprising the oligonucleotides.

The term "anti-miR" means an oligonucleotide having a sequence complementary to a microRNA. It may be a modified oligonucleotide.

The term "anti-miR-335" means an oligonucleotide having a sequence complementary to miR-335. It may be a modified oligonucleotide.

In this context, antimirs can be designed to a large region of the mature miR (e.g., 16 mer) or the entire mature miR.

The term "miR-mimic" is an RNA fragment that mimics endogenous miRNA. It can bind to its target gene and produce posttranscriptional repression, more specifically translational inhibition, of the gene.

The term "subject in need thereof" is a subject that is identified as in need of a therapy or treatment.

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The term "neurological sodium channelopathy" is a sodium channelopathy that affects the brain as well as the nerves found throughout the body and the spinal cord. Preferably, the channelopathy is a brain disorder.

The term "neurodevelopmental disorder" is a condition associated primarily with the development and functioning of the neurological system and the brain. Such disorders typically affect behaviour, memory and/or ability of learn and/or motor abilities and/or speech and/or mood.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

In its broadest sense the invention provides a modulator of miR-335 for use in the treatment or prevention of a sodium channelopathy in a subject. miR-335 is differentially expressed in different disease states. The inventors show experimental manipulation of mirRNA-335 using a miR-335 modulator of the invention can alter the expression of these genes and, ultimately, modulate brain excitability.

For sodium channelopathies which are neurological channelopathies the modulator of the invention may treat the seizure by lessening the seizure and/or reducing the severity of the seizure. A reduction in the number of seizures is provided. The comorbidities may also be improved. For channelopathies that are not associated with a seizure, such as non-epileptic conditions, e.g., pain, the treatment would be a reduction in pain.

The disease or condition to be treated or prevented may be a neurological condition associated with a mutation in one or more voltage-gated sodium channel gene, or genes, a sodium leak channel gene or genes, or in UBE3A.

The sodium channelopathy is a disease or condition caused by or associated with a mutation a sodium channel gene or genes. The sodium channel may be a voltage-gated sodium channel or a sodium leak channel. The gene may be one or more of SCN1A, SCN2A, SCN3A, SCN4A, SCN9A, SCN8A or NALCN.

The mutation may be a loss of function mutation, or a gain of function mutation.

Notably, the mutation in the voltage-gated sodium channel may be a loss of function mutation. In this instance, the modulator is an inhibitor of miR-335.

The sodium channelopathy may be one associated with a loss of function mutation in one or more of SCN1A, SCN2A, SCN3A, SCN9A, SCN8A and SCN4A.

Examples of SCN1A sodium channelopathies with a loss of function mutation in SCN1A include Dravet syndrome, developmental and epileptic encephalopathy 6, generalised epilepsy with febrile seizures plus type 2 and severe myoclonic epilepsy of infancy (SMEI). In such cases, the modulator is one that inhibits miR-335.

Examples of SCN2A sodium channelopathies with a loss of function mutation in SCN2A include autism and epilepsy, such as rare childhood epilepsy and channelopathies with late seizure onset phenotype, ASD, and intellectual disability with later onset mild epilepsy or without epilepsy. The inventors have shown that antagonising or inhibiting miR-335 activity, e.g., with anti-miR-335, has upregulated SCN2A, which is a gene that enclosed voltage gated sodium channel Nav1.2. This has a therapeutic implication for diseases where SCN2A loss of function predominates, such as autism and rare childhood epilepsies. In such cases, the modulator is one that inhibits miR-335.

Some cases of Dravet syndrome may also be associated or caused by a loss of function mutation in SCN2A.

An example of SCN3A sodium channelopathy with a loss of function mutation in SCN3A is a disease associated with p.Leu247Pro mutation in Nav1.3, such as severe infantile onset epileptic encephalopathy.

Examples of SCN9A sodium channelopathy with a loss of function mutation in SCN9A include complete insensitivity to pain (CIP), anosmia (loss of sense of smell), and primary erythromelalgia (PEM).

An example of an SCN8A sodium channelopathy with a loss of function mutation in SCN8A is ataxia.

Notably, the mutation in the voltage-gated sodium channel may be a gain of function mutation. The sodium channelopathy may be one associated with a gain of function mutation in one or more of SCN1A, SCN2A. SCN3A, SCN9A, SCN8A and SCN4A. In such cases, the modulator is one that increases miR-335.

For instance, the current inventors have shown that increasing the activity of miR-335 has a therapeutic effect in SCN1A associated epilepsies.

Examples of SCN2A sodium channelopathies with a gain of function mutation in SCN2A include epilepsy, preferably developmental and epileptic encephalopathy 11 (DEE11), episodic ataxia type 9 (EA9), benign familial infantile seizures-3 (BFIS3), and Ohtahara syndrome.

Examples of SCN1A sodium channelopathies with a gain of function mutation in SCN1A include familial hemiplegic mirgraine-3 and a disease associated with T226M NaV.1.1 mutation, such as developmental and early infantile epileptic encephalopathy.

Examples of SCN3A sodium channelopathies with a gain of function mutation in SCN3A include developmental and epileptic encephalopathy 62, familial focal epilepsy with variable foci-4 and infantile onset epileptic encephalopathy.

Examples of SCN9A sodium channelopathies with a gain of function mutation in SCN9A include paroxysmal extreme pain disorder (PEPD) and inherited erythromelalgia (IEM).

Voltage-gated ion channels are essential for the generation and propagation of action potentials, chiefly in nerve and muscle. Voltage-sensitive sodium channels are heteromeric complexes consisting of a large central pore-forming glycosylated alpha subunit and 2 smaller auxiliary beta subunits.

SCN2A is sodium voltage-gated channel, alpha subunit 2. It enclosed the voltage gated sodium channel Na(v)1.2, which plays an important role in the initiation and conduction of action potentials. It has the following nucleic acid sequence in humans and the following amino acid sequence. It is located on chromosome 2q24 in humans. An exemplary sequence for human SCN2A gene (and the protein) can be found at Ensembl ENSG00000144285. It will be appreciated that variants of the gene sequence (or protein sequence), including fragments may occur in a species and all such are included in the current method.

The SCN2A sodium channelopathy may be one or more selected from the group comprising developmental and epileptic encephalopathy 11 (DEE11), episodic ataxia type 9 (EA9), benign familial infantile seizures-3 (BFIS3). Gain of function mutations in SCN2A are associated with early seizure onset phenotype.

Developmental and epileptic encephalopathy-11 (DEE11) is a neurologic disorder characterized by onset of seizures in the first days, weeks, or months of life. Some patients may have later onset. Seizures comprise multiple types, including tonic, generalized, and myoclonic, and tend to be refractory to medication. It is associated with a heterozygous mutation in the SCN2A gene. In addition to DEE11 the subject may have benign familial infantile seizures-3 (BFIS3) whereas others may be episodic ataxia type 9 (EA9).

Episodic ataxia type 9 (EA9) is a neurologic disorder characterized by onset of ataxic episodes in the first years of life. Features may include difficulty walking, dizziness, slurred speech, headache, vomiting, and pain. It is associated with a heterozygous mutation in the SCN2A gene.

Benign familial infantile seizures-3 (BFIS3) is an autosomal dominant disorder in which afebrile seizures occur in clusters during the first year of life, without neurologic sequelae. It is associated with a heterozygous mutation in the SCN2A gene.

Autism is a prototypic pervasive developmental disorder (PDD) and is usually apparent by 3 years of age. It is characterized by a triad of limited or absent verbal communication, a lack of reciprocal social interaction or responsiveness, and restricted, stereotypic, and ritualized patterns of interests and behavior (Bailey et al., 1996; Risch et al., 1999). 'Autism spectrum disorder,' sometimes referred to as ASD, is a broader phenotype encompassing the less severe disorders, including Asperger syndrome and pervasive developmental disorder, not otherwise specified (PDD-NOS). 'Broad autism phenotype' includes individuals with some symptoms of autism, but who do not meet the full criteria for autism or other disorders. Mental retardation coexists in approximately two-thirds of individuals with ASD, except for Asperger syndrome, in which mental retardation is conspicuously absent (Jones et al., 2008). Jiang et al 2013, (Detection of clinically relevant genetic variants in autism spectrum disorder by whole-genome sequencing. Am. J. Hum. Genet. 93: 249-263, 2013) reported SCN2A as associated with ASD risk. Tavassoli et al., 2014, (De novo SCN2A splice site mutation in a boy with autism spectrum disorder. BMC Med. Genet. 15: 35, 2014) identified de novo heterozygous splice mutations in the SCN2A gene.

SCN1A is sodium voltage-gated channel, alpha subunit 1, and encodes NaV1.1. SCN1A sodium channelopathies may be selected from the group comprising developmental and epileptic encephalopathy 6B (DEE6B), Dravet Syndrome, Generalised epilepsy with febrile seizures plus type 2 (GEFSP2) and Familial hemiplegic migraine-3 (FHM3). Gain of function mutations in SCN1A are associated with epilepsy phenotypes. An exemplary sequence for human SCN1A gene (and the protein) can be found at Ensembl ENSG00000136531. It will be appreciated that variants of the gene sequence (or protein sequence), including fragments may occur in a species and all such are included in the current method.

Developmental and epileptic encephalopathy-6B (DEE6B) is a severe neurodevelopmental disorder characterized by early-infantile seizure onset, profoundly impaired intellectual development, and a hyperkinetic movement disorder. Brain imaging usually shows progressive atrophy and other abnormalities (Sadleir et al., Not all SCN1A epileptic encephalopathies are Dravet syndrome: early profound thr226met phenotype. Neurology 89: 1035-1042, 2017).

Dravet syndrome is a clinical term for a severe neurological disorder characterised by the onset of seizures in the first year of life after normal early development. Affected individuals usually present with generalized tonic, clonic, and tonic-clonic seizures that may initially be induced by fever and are usually refractory to treatment. Later, patients tend to manifest other seizure types, including absence, myoclonic, and partial seizures. Most cases of Dravet syndrome are caused by mutation in the SCN1A gene.

Generalised epilepsy with febrile seizures plus type 2 (GEFSP2) is an autosomal dominant neurologic disorder characterized by the onset of seizures associated with fever in the first months or years of life. Affected individuals continue to have various types of febrile and afebrile seizures later in life, including generalized tonic-clonic seizures (GTCS). Some patients may have offset of seizures in the first or second decades; rare patients may have mildly impaired intellectual development. It is caused by a mutation in the SCN1A gene. (Scheffer, I. E., et al., Generalized epilepsy with febrile seizures plus: a genetic disorder with heterogeneous clinical phenotypes. Brain 120: 479-490, 1997).

Familial hemiplegic migraine-3 (FHM3) is a severe subtype of migraine with aura characterized by some degree of hemiparesis during the attacks (Dichgans, M., et al., Mutation in the neuronal voltage-gated sodium channel SCN1A in familial hemiplegic migraine. Lancet 366: 371-377, 2005). FHM3 is caused by heterozygous mutations in the SNC1A gene.

The SCN3A gene encodes the alpha subunit of the voltage-gated sodium channel NaV1.3, which shows rapid activation and inactivation. SCN3A sodium channelopathies may be selected from the group comprising developmental and epileptic encephalopathy-62 (DEE62) and Familial focal epilepsy with variable foci-4 (FFEVF4). Each of these channelopathies is associated with a gain of function mutation in SCN3A. Gain of function mutations in SCN1A are associated with epilepsy phenotypes. An exemplary sequence for human SCN3A gene (and the protein) can be found at Ensembl ENSG00000153253. It will be appreciated that variants of the gene sequence (or protein sequence), including fragments may occur in a species and all such are included in the current method.

Developmental and epileptic encephalopathy-62 (DEE62) is a severe neurologic disorder characterized by the onset of various types of refractory seizures in the first weeks or months of life. Affected individuals have severe to profound developmental delay with hypotonia and impaired motor and cognitive development. Additional features may include spasticity, microcephaly, and brain imaging abnormalities (Zaman, T., et al., Mutations in SCN3A cause early infantile epileptic encephalopathy. Ann. Neurol. 83: 703-717, 2018). DEE62 is caused by heterozygous mutations in the SCN3A gene on chromosome 2q24.

Familial focal epilepsy with variable foci-4 (FFEVF4) is an autosomal dominant seizure disorder characterized by onset of focal seizures in the first years of life. Some patients may have secondary generalization and/or mild developmental deficits (Vanoye, C. G., Gurnett, C. A., Holland, K. D., George, A. L., Jr., Kearney, J. A. Novel SCN3A variants associated with focal epilepsy in children. Neurobiol. Dis. 62: 313-322, 2014). FFEVF4 is caused by heterozygous mutations in the SCN3A gene on chromosome 2q24.

SCN9A encodes a voltage-gated sodium channel that is enriched in nociceptive and sympathetic neurons of the peripheral nervous system, namely NaV1.7. It is also expressed in subcortical structures of brain (McDermott, L. A., et al., Defining the functional role of NaV1.7 in human nociception. Neuron 101: 905-919, 2019). SCN9A sodium channelopathies may be selected from the group comprising primary erythermalgia, congentical insensitivity to pain, peripheral pain, neuropathy hereditary sensory and autonomic type IID, paroxysmal extreme pain disorder and small fiber neuropathy. Peripheral pain is associated with a gain of function mutation in SCN9A. An exemplary sequence for human SCN9A gene (and the protein) can be found at Ensembl ENSG00000196876. It will be appreciated that variants of the gene sequence (or protein sequence), including fragments may occur in a species and all such are included in the current method.

SCN4A encodes a sodium voltage gated channel, alpha subunit 4, namely NaV1.4. SCN4A sodium channelopathies may be selected from the group comprising hyperkalemic periodic paralysis type 2, hypokalemic periodic paralysis type 2, myasthenic syndrome congenital 16, myotonia congenita (atypical) acetazolamide-responsive and paramyotonia congenita. An exemplary sequence for human SCN4A gene (and the protein) can be found at Ensembl ENSG00000007314. It will be appreciated that variants of the gene sequence (or protein sequence), including fragments may occur in a species and all such are included in the current method.

Human temporal lobe epilepsy (TLE). SCN4B is dysregulated in drug resistant human temporal lobe epilepsy. MiR-335 can treat TLE by suppressing SCN2A, and/or SCN3A and/or SCN4B. TLE may be treated with an miR-335 modulator capable of increasing miR-335.

SCN8A is voltage-gated channel alpha subunit 8 (Nav1.6). An exemplary sequence for human SCN8A gene (and the protein) can be found at Ensembl ENSG00000196876. It will be appreciated that variants of the gene sequence (or protein sequence), including fragments may occur in a species and all such are included in the current method.

The following table provides the SCN gene mutation type associated with the channelopathy listed.

**Table 1: SCN mutations present in sodium channelopathies. LOF=Loss of function; GOF=gain of function.**

| Sodium channelopathy | SCN gene | LOF or GOF |
|---|---|---|
| benign familial infantile seizures-3 | SCN2A | GOF |
| developmental and epileptic encephalopathy 11 | SCN2A | GOF |
| episodic ataxia type 9 | SCN2A | GOF |
| Ohtahara syndrome | SCN2A | GOF |
| ASD or intellectual disability with later-onset mild epilepsy or without epilepsy | SCN2A | LOF |
| Dravet Syndrome | SCN1A | LOF |
| developmental and epileptic encephalopathy 6B | SCN1A | LOF |
| Generalised epilepsy with febrile seizures plus type 2 | SCN1A | LOF |
| Familial hemiplegic migraine-3 | SCN1A | GOF |
| severe myoclonic epilepsy of infancy (SMEI) | SCN1A | LOF |
| A disease associated with T226M Naᵥ1.1 mutation | SCN1A | GOF |
| developmental and epileptic encephalopathy-62 | SCN3A | GOF |
| Familial focal epilepsy with variable foci-4 | SCN3A | GOF |
| infantile onset epileptic encephalopathy | SCN3A | GOF |
| p.Leu247Pro mutation in Nav1.3 | SCN3A | LOF |
| ataxia | SCN8A | LOF |
| complete insensitivity to pain (CIP) | SCN9A | LOF |
| Anosmia (loss of sense of smell) | SCN9A | LOF |
| primary erythromelalgia (PEM) | SCN9A | LOF |
| Paroxysmal extreme pain disorder (PEPD, initially known as familial rectal pain), | SCN9A | GOF |
| inherited erythromelalgia (IEM) | SCN9A | GOF |

UBE3A (ubiquitin-protein ligase E3A) functions as a E3 ligase in the ubiquitin proteasome pathway and as a transcriptional coactivator. UBE3a is located on chromosome 15. In the brain, UBE3 is inactivated on the paternal allele and active on the maternal allele. Any loss of function to the maternal copy results in loss of the gene's expression which is the case in Angelman's syndrome. Mutations directly in the UBE3a gene lead to an abnormally short, non-functional version of ubiquitin protein ligase E3A. In addition, chromosomal changes of chromosome 15 as rearrangement of genetic material or defect in the DNA of the UBE3A gene prevent any functional form of ubiquitin protein ligase E3A. For Prader-Willi Syndrome the paternal copy of the UBE3A gene is affected. An exemplary sequence for human UBE3A gene (and the protein) can be found at Ensembl ENSG00000114062. It will be appreciated that variants of the gene sequence (or protein sequence), including fragments may occur in a species and all such are included in the current method.

Angelman syndrome is a neurodevelopmental disorder characterized by mental retardation, movement or balance disorder, typical abnormal behaviours, and severe limitations in speech and language. Most cases are caused by absence of a maternal contribution to the imprinted region on chromosome 15q11-q13. A subset is caused by mutations in UBE3A.

The modulator may be an inhibitor. The modulator may be increase miR-335.

When there is a loss of function mutation, typically the modulator is an inhibitor of miR-335.

The sodium leak channel (non-selective) is encoded by the NALCN gene. The channelopathy may be one associated with a gain of function mutation or a loss of function mutation in NALCN. NALCN forms a voltage-independent, nonselective, non-inactivating cation channel permeable to Na+, K+, and Ca(2+). It is responsible for the neuronal background sodium leak conductance; mainly responsible for the leaky sodium transport across neuronal membranes and controls neuronal excitability (Lu et al., 2007). The NALCN gene is located on chromosome 13q33.1

The NALCN ion channel forms a channelosome (Channel complex) which is an interaction between several proteins (Cochet-Bissuel, et al., The sodium leak channel, NALCN, in health and disease, Front. Cell. Neurosci, May 2014). Genes, which are involved in the NALCN channelosome, are NALCN, UNC-80, UNC-79, NLF-1, CHRM3 and SCR. Autosomal Recessive Syndrome with severe hypotonia, speech impairment, and cognitive delay, is cause by a LOF mutation in NALCN. Infantile Neuroaxonal Dystrophy (INAD) is caused by a LOF function mutation in NALCN. The identified mutation was a C to T conversion in *NALCN* coding exon 16 (c.1924C > T), creating a premature translational termination signal at codon 642 (Q642X) and truncated NALCN channel.

NALCN mutations also play a role in epilepsy. Patients with INAD or an autosomal-recessive syndrome with severe hypotonia, speech impairment, and cognitive delay have seizures (Al-Sayed et al., 2013; Koroglu et al., 2013). Seizures were also reported for patients with chromosome 13q deletions in regions that contain the *NALCN* gene (Kirchhoff et al., 2009; Lalani et al., 2013). An exemplary sequence for human NALCN gene (and the protein) can be found at Ensembl ENSG00000102452. It will be appreciated that variants of the gene sequence (or protein sequence), including fragments may occur in a species and all such are included in the current method.

miR-335 modulators include those that inhibit miR-335. These include agents that inhibit miR-335 as defined herein. Agents that inhibit miR-335 may be those known in the art. An antisense oligonucleotide of SEQUENCE ID NO. 4 is one example of a modulator of the invention.

Examples of antagomirs are provided in Shu, M., et al. Targeting oncogenic miR-335 inhibits growth and invasion of malignant astrocytoma cells. Mol Cancer 10, 59 (2011).

miR-modulators also include gene therapy methods. It will be appreciated that gene therapy methods are known in the art and could be used to repress the expression level of miR-335. The gene therapy method could be used to express any of the inhibitors/repressors of miR-335 levels/expression/activity in the subject. These may be provided on a delivery vector, such as a viral vector. One example is AAV9. It can be targeted to specific cell or tissue types

Inhibition of miR-335 should also be understood to include an agent that is a target site blocker (TSB), that specifically disrupts the binding of miR-335 at the 3' UTR site of the target mRNA, i.e., the mRNA for one or more of SCN1A, SCN2A. SCN3A, SCN9A, SCN8A and SCN4A, UBE3A or NALCN. For example, it could be an agent that is designed to bind to one or more miR-335 mRNA binding sites to selectively block miRNA-335 activity. This allows a more selective therapeutic approach rather than blocking all activity via an miR-335 antagomir for example.

miR-335 modulators include those that increase or enhance miR-335. For instance, relative to the level of activity of human miR-335 in the subject that is not contacted with or prior to contact with the modulator. Agents that increase or enhance miR-335 may be those known in the art. These include but are not limited to miR-335 mimics and miR-335. These may be provided on a delivery vector. Preferably, the vector is a viral vector. One example is AAV9. The vector may be formulated or provided for tissue or cell specific delivery. It will be appreciated that gene therapy methods are known in the art and could be used to increase the expression level of miR-335. The gene therapy method could be used to express any of the enhancers of miR-335 levels/expression/activity.

Thermofisher scientific provides human and mouse miR-335 inhibitors and mimics. For example, mirVana^{®} miRNA inhibitor, mirVana^{®} miRNA mimic and anti-miR^{™} miRNA inhibitor (thermofisher.com). Further examples are found at www.ribobio.com.

miR-335 may be provided in the form of miR-335 expressing cells.

In an aspect of the invention there is provided a method of identifying compounds useful in the treatment or prevention of a sodium channelopathy, comprising contacting miR-335 with a candidate compound and determining the level of activity of the contacted miR-335 RNA. A decrease in the level of activity of the contacted miR-335 RNA relative to a reference level of activity of human miR-335 that is not contacted with the compound is an indication that the candidate compounds is a useful miR-335 inhibitor and thus suitable to treat or prevent a sodium channelopathy which requires a decrease or inhibition of miR-335. An increase in the level of activity of the contacted miR-335 RNA relative to a reference level of activity of human miR-335 that is not contacted with the compound is an indication that the candidate compounds is useful to increase miR-335 and thus suitable to treat or prevent a sodium channelopathy which requires an increase of miR-335.

In one embodiment of the present invention, the miR-335 RNA is provided in the form of miR-335 expressing cells, and in which the level of activity is determined by assaying for a level of expression of miR-335 RNA in the cells.

Preferably, the target tissue is the brain of the subject. It may be direct delivery or indirect delivery. The delivery method may be intrathecal, intranasal, intracerebroventricular, systemic or intracerebral delivery. The composition or modulator of the invention may be formulated such that it is suitable for the specific delivery method.

Various delivery systems are known and can be used to administer the modulator or composition of the invention, e.g., intra-nasally. Methods of introduction include but are not limited to intradermal, intramuscular, intraperitoneal, intravenous, subcutaneous, intranasal, epidural, intranasal, intracerebral, and oral routes. The compositions may be administered by any convenient route, for example by infusion or bolus injection, by absorption through epithelial or mucocutaneous linings (*e.g.,* oral mucosa, rectal and intestinal mucosa, *etc.*) and may be administered together with other biologically active agents. Administration can be systemic or local. In addition, it may be desirable to introduce the compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

Preferably, the therapeutic is delivered to the CNS or PNS. Delivery means include intravenous delivery, oral delivery, intramuscular delivery, intrathecal delivery, and inhaled delivery. Methods for achieving these means of delivery will be well known to those skilled in the art of drug delivery, and include:
- Delivered intrathecially by mini-osmotic pump. (ref: Ignacio et al., Ann. N.Y. Acad. Sci. 2005, 1053: 121-136).
- Intramuscular delivery directly into muscle(s) by syringe or mini osmotic pump (Azzouz et al., Nat Med. 2005;11(4):429-33).
- Intraperitoneal- for systemic administration - directly administered to peritoneum by syringe or mini osmotic pump (Kieran et al., Nat Med 2004; 10(4):402).
- Subcutaneous- for systemic administration - directly administered below the skin by syringe (Reinholz et al., Exp Neurol. 1999;159(1):204-16).
- Intraventricular- direct administration to the ventricles in the brain, by injection or using small catheter attached to an osmotic pump.(Sathasivam et al., 2005 Neuropath App Neurobiol; 31(5): 467)
- Implant- can be prepared in an implant (eg small silicon implant) that will release Active. Implant can be placed at muscles or directly onto the spinal cord (Kieran and Greensmith, 2004 Neurosci 125(2):427-39).

It may be desirable to administer the modulator or compositions of the invention locally to the area in need of treatment; this may be achieved, for example and not by way of limitation, by topical application, by injection, by means of a catheter, by means of a suppository, or by means of an implant, said implant being of a porous, non-porous, or gelatinous material, including membranes, such as sialastic membranes, or fibers.

The present invention also provides pharmaceutical compositions. Such compositions comprise a therapeutically effective amount of the therapeutic, and a pharmaceutically acceptable carrier.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, *etc.* Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The dose and frequency of the dose may be any suitable dose and frequency depending on the subject and/or the severity of the disease. It will be appreciated that the particular dose will vary depending on the subject, the age and general condition of the subject, mode of administration and other factors. While it is not possible to specify an exact dose and frequency, those skilled in the art will be able to determine an appropriate dose and frequency in any individual case using routine experimentation and background general knowledge.

The modulator or composition of the invention may be administered in combination with one or more therapies for treatment or prevention of sodium channelopathy. This includes any such therapies known and used in the art for the conditions.

As aspect of the invention also provides a miR-335 modulator of the invention for modulating the expression levels of a sodium channel gene selected from the group comprising SCN1A, SCN2A. SCN3A, SCN8A, SCN9A, SCN4A, and NACLN. Preferably, the gene is one or more of SCN1A, SCN2A. SCN3A, SCN8A, SCN9A and SCN4A.

As aspect of the invention also provides a miR-335 modulator of the invention for modulating the expression levels UBE3A.

The invention further provides a method for modulating the expression levels of one or more sodium channel genes selected from the group comprising SCN1A, SCN2A. SCN3A, SCN8A, SCN9A, SCN4A, and NACLN. The method comprises contacting said one or more genes with a miR-335 modulator of the invention.

The invention further provides a method for modulating the expression levels of UBE3A. The method comprises contacting UBE3A with a miR-335 modulator of the invention.

The invention further provides a method for treating or preventing a sodium channelopathy in a subject, said method comprising administering an amount, such as a therapeutically effective amount, of the miR-335 modulator of the invention to said subject.

The invention further provides a method for treating or preventing a UBE3A associated condition, said method comprising administering an amount, such as a therapeutically effective amount, of the miR-335 modulator of the invention to said subject.

It will be appreciated that the features and aspects of the use of the invention may also apply to the methods of the invention.

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLES

### Materials and Methods

### In vivo experiments

### Animals and husbandry

All experimental procedures involving animals were carried out in accordance with the European Communities Council Directive (2010/63/EU) and were reviewed and approved by the Research Ethics Committee of the Royal College of Surgeons in Ireland (RSCI) (REC # 1587 and 1302), under licenses from the Health Products Regulatory Authority (HPRA), Dublin, Ireland (AE19127/P057 and AE19127/P064).

Male C57BL/6JOIaHsd mice were originally obtained from Harlan Laboratories (Bicester, UK). Scn1a mutant mice were obtained from Jackson Laboratory (Maine, US) and bred as described below. All mice were bred in house in the Biomedical Research Facility (BRF) in RCSI. All surgical and behavioural procedures were performed in the Microsurgical Research Training Facility (MRTF) in RCSI. Mice under P21 were housed with their littermates. Weaned animals were group housed (2-5/cage) unless otherwise indicated. All animals were housed on a 12 h/12 h light/dark cycle under controlled conditions (temperature: 20-25°C; humidity: 40-60%). Food and water were available ad libitum.

### Surgical procedures

Surgeries were performed on WT C57BI/6J P19/P21 and adult male mice for the majority of the experiments.

### Intracerebroventricular (i.c.v.) injections of miR-335 inhibitor

Mice were anaesthetised with 5% isoflurane in oxygen and placed in a stereotaxic frame. Anaesthesia through isoflurane was maintained at 2-2.5% in oxygen during the time of the surgery. Then, the head of the animal was fixed in place and local anaesthetic (bupivacaine (3 mg/kg; s.c.) and EMLA cream) and iodine (Videne, Ecolab Ltd., UK) was applied. A midline scalp incision was performed and the skin was folded away to expose the skull. Bregma was located, the head was aligned and a partial craniotomy was drilled to allow the direct injection into the right lateral ventricle (coordinates from Bregma: A/P = +0.3 mm; L = -0.9 mm; dorso-ventral; (D/V) = -2 mm, Fig. 1). Mice received either miRCURY LNA power inhibitor for mmu-miR-335-5p (Ant-335; sequence: 5'-CATTTTTCGTTATTGCTCTTG-3 (SEQUENCE ID NO. 4)'; Qiagen; 0.05-0.5 nmol in 2 µl PBS), or a non-targeting scrambled control (Scr; sequence: 5'-TAACACGTCTATACGCCCA-3'(SEQUENCE ID NO. 5); Qiagen; 0.1-0.5 nmol in 2 µl PBS), using a 2 µl Hamilton syringe at a rate of 1 µl/min. miRCURY LNA miRNA Inhibitors are antisense oligonucleotides with perfect sequence complementary to their targets. To ensure even distribution in the ventricle and to avoid backflow, the i.c.v. needle was left in the brain for 5 minutes after the injection. After, the needle was slowly removed from the brain and the surgical site was closed with surgical glue (Surgibond, SMI AG, Belgium).

After surgery, animals were placed in a temperature-controlled 25°C incubator and monitored for post-surgery recovery.

### Intra-hippocampal injections of viral vectors

Mice were anaesthetised with 5% isoflurane in oxygen and placed in a stereotaxic frame. Anaesthesia through isoflurane was maintained at 2-2.5% in oxygen during the time of the surgery. Then, the head of the animal was fixed in place and local anaesthetic (bupivacaine (3 mg/kg; s.c.) and EMLA cream) and iodine (Videne, Ecolab Ltd., UK) was applied. A midline scalp incision was performed and the skin was folded away to expose the skull. Bregma was located, the head was aligned and two partial craniotomies were drilled to allow the direct injection into the left and right ventral hippocampus (coordinates from Bregma: A/P = -3 mm; L = ±2.62 mm; D/V: -2.5/ -3/ -3.5 mm from pia; figure 2.2A). Injections were administered using a 2 µl Hamilton syringe placed in a syringe pump (Pump 11 Elite, Harvard Apparatus, MA, USA) at a rate of 0.1 µl/min and 0.2ul was injected per injection site. Mice received either AAV9-SYN1-EGFP-mmu-miR335-5p (AAV9-miR-335; VectorBuilder Inc., Fig. 2B) or AAV9-SYN1-EGFP-Scramble (AAV9-Scr; VectorBuilder Inc., Fig. 2C). To ensure even distribution in the ventricle and to avoid backflow, the needle was left in the brain for 2 minutes after the injection. After, the needle was slowly removed from the brain and the surgical site was closed with surgical glue (Surgibond, SMI AG, Belgium).

After surgery, animals were placed in a temperature-controlled 25°C incubator and monitored for post-surgery recovery.

### Induction of seizures using pentylenetetrazol (PTZ)

To induce acute generalised seizures, adult C57BL/6J male mice received a convulsant dose of 75-80 mg/kg of PTZ (Sigma-Aldrich, UK) in 0.9% (w/v) NaCl (B.Braun Medical Limited, Ireland) via an i.p. injection. Mice were placed individually in a clear chamber and video monitored for 30 minutes to record their behaviour. After the observation period, animals were directly euthanised.

Videos of PTZ-induced seizures were analysed offline using a modified Racine scale: 0, no change in behaviour; 1, isolated myoclonic jerks; 2, atypical clonic seizure; 3, fully developed bilateral forelimb clonus; 4, tonic-clonic seizures with suppressed tonic phase with loss of righting reflex; 5, fully developed tonic-clonic seizure with loss of righting reflex (Pohl and Mares, 1987). For each animal, the latency (in seconds) from the PTZ injection to the development of a first sign of seizure, clonic seizure and tonic-clonic seizure and the maximal seizure severity were recorded. Additionally, the percentage of animals that experienced the highest score and the mortality rate for each group were calculated.

### Transcardial perfusion and tissue preparation

After an anaesthetic overdose with sodium pentobarbital (i.p.; Dolethal, Vetoqinol, UK), mice were transcardially perfused with 1x PBS to remove intravascular blood components. For histology, brains were directly flash frozen in 2-methylbutane on dry ice. For molecular analysis, whole brain was removed from the skull and placed on small petri dish on top of an ice block. The cerebellum was then dissected away and the two hemispheres were separated along the longitudinal fissure. After the removal of the midbrain, hippocampi and cortices of both hemispheres were collected. Tissue was directly frozen on dry ice and stored until used at -80°C.

### In silico analysis

### miRNA target interaction (MTI) analysis

To identify predicted miRNA targets, the database miRDiP V4.1 was used. This database integrates 30 prediction algorithms and calculates a miRNA target interaction (MTI) confidence score based on statistical inference (Tokar et al., 2018). Experimentally validated targets for differently expressed miRNAs were obtained from miRTarbase V7 (Chou et al., 2018) and TarBase V7.0 (Karagkouni et al., 2017). To ensure the compatibility of all miRNAs, miRNA names were uploaded to miRNAmeConverter (Haunsberger et al., 2017) and translated to miRBase V22. Enseml V91 was used to convert all mRNA names to official gene symbols. Non-human MTIs were excluded to limit analyses to putative translatable mechanisms.

Epilepsy related miRNA targets were identified using an in-house database collecting information from CARPEDB [http://carpedb.ua.edu], epiGAD (Tan & Berkovic, 2010), Wang et al. (Wang et al., 2017), and curated epilepsy genes from the Comparative Toxicogenomics Database (CTD; Davis et al., 2019). MTI analysis was performed by Dr Niamh Connolly. The miRNA-target interaction network was generated using Cytoscape. Only epilepsy related targets were uploaded to Cytoscape to generate the interaction network.

### Molecular work

### Total RNA extraction

### RNA extraction from brain tissue

Total RNA (mRNAs + miRNAs) was extracted from the right (ipsilateral) hippocampus using the Trizol method. Briefly, the tissue was homogenised in 800 µl Trizol (Qiagen, UK), followed by centrifuging samples at 12,000 g for 10 minutes at 4°C. The supernatant was transferred to a new tube and incubated for 5 minutes at room temperature. 200 µl of chloroform (Sigma-Aldrich, Ireland) was added to the supernatant, shaken shortly and then incubated for 3 minutes at room temperature before being spun at 16,200 g for 15 minutes at 4°C. Finally, the upper aqueous phase was transferred to a fresh tube and 450 µl or greater (depending on the volume of the aqueous phase) isopropanol (Sigma-Aldrich, Ireland) and 1.5 µl Glycoblue (Invitrogen, Ireland) were added. The samples were incubated at -20°C overnight.

On the second day, samples were centrifuged at maximum speed (17,000 g) for 30 minutes at 4°C. The supernatant was discarded and the pellet washed with 400 µl of cold 75% ethanol before being centrifuged again at maximum speed for 10 minutes at 4°C. The washing step was repeated and then the pellet allowed to air dry completely. To measure the quantity and quality of RNA, the pellet was resuspended in 15-25 µl dH₂O and measured using a Nanodrop Spectrophotometer (Thermo Fisher Scientific, Ireland). Samples with an absorbance ratio at 260 nm/280 nm between 1.8-2.2 were considered as acceptable.

### Concentration and purification of RNA samples

To concentrate and purify the RNA content, 100% ethanol, 3 M sodium acetate (Invitrogen, Ireland) and Glycoblue (Invitrogen, Ireland) were added to the RNA samples and stored overnight at -20°C. On the next day, samples were centrifuged at maximum speed for 30 minutes at 4°C. Supernatants were discarded and the pellet washed with cold 75% ethanol. Samples were centrifuged again at maximum speed for 10 minutes at 4°C. Supernatants were removed and the pellet was allowed to air dry. After, RNA pellets were resuspended in 10-20 µl dH₂O and measured using the Nanodrop Spectrophotometer.

### Analysis of individual miRNA expression

### Reverse Transcription (RT) PCR

For the reverse transcription step, 250 ng of total RNA was diluted in RNase-free water and made up to a 5 µl in 200 µl PCR. A master mix containing 0.15 µl dNTPs, 0.19 µl RNase Inhibitor, 1 µl Multiscripe RT enzyme, 1.5 µl 10x RT buffer and 5.16 µl dH₂O was prepared. 8 µl of master mix and 2 µl of miRNA-specific RT primer (Applied Biosystems, Dublin, Ireland, table 2) were added to the diluted RNA. Samples were briefly centrifuged and then incubated under the listed PCR conditions (table 3).

**Table 2: miRNA primers and sequences used**

| **Primer** | **Sequence** | |
|---|---|---|
| miR-335 | UCAAGAGCAAUAACGAAAAAUGU | |
| U6B | (SEQ ID NO. 6) CGCAAGGATGACACGCAAATTCGTGAAGCGTTCCATATTTTT | |

**Table 3: Reverse transcription PCR cycles**

| ₁ **Step** | **Temperature (°C)** | **Incubation time** |
|---|---|---|
| | 16 | 30 min |
| 2 | 42 | 30 min |
| 3 | 85 | 5 min |
| 4 | 4 | ∞ |

### Quantitative PCR

Quantitative PCR (qPCR) was performed using the QuantStudio^{™} Flex PCR system (Thermo Fisher Scientific, Ireland). Generated cDNA was diluted with nuclease-free dH₂O in a ratio of 1:10. 1 µl of diluted cDNA was then mixed with a master mix containing 5 µl 2x TaqMan Fast Universal PCR Master Mix, 0.5 µl miRNA-specific PCR primers and 3.5 µl of dH₂O. Samples were added in triplicates in a 96-well plate. The plate was then covered with an optical adhesive film (MicroAmp, Applied Biosystems) and briefly centrifuged at 1000 g for 1 minute and placed in the QuantStudio^{™} 12K Flex PCR system. qPCR was then performed under the cycle conditions listed in table 4. Comparative CT values were measured. MiRNA levels were normalised using U6B expression and relative fold change in miRNA levels were calculated using the comparative cycle threshold method (2-ΔΔCT).

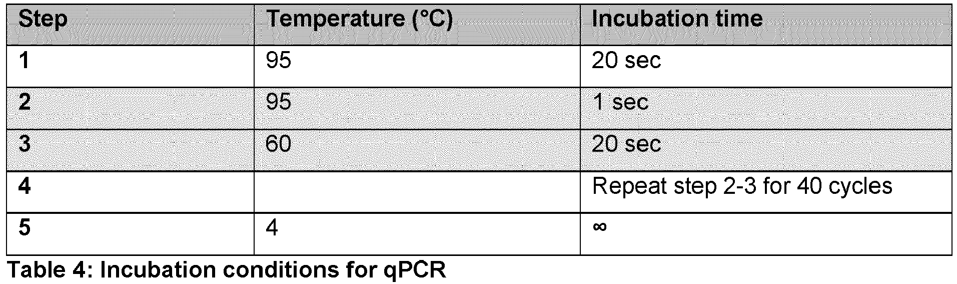

### Analysis of individual gene expression

### Reverse Transcription (RT) PCR

For the reversed transcription step, 500 ng of total RNA was diluted in RNase free water and made up to 8 µl. For the degradation step, 1 µl of DNase 10x buffer and 1 µl of DNase I was added to the diluted RNA sample and incubated for 15 minutes at room temperature. After, the reaction was stopped by adding 1 µl EDTA (25 mM) and samples were incubated at 65°C for 10 minutes. Then, 1 µl of random hexamer (Invitrogen, Ireland) primers were added to each sample. The reversed transcription process to obtain cDNA was started by using the program below (table 5). First, samples were incubated at 65°C for 5 minutes followed by a cooling step at 4°C for 1 minute. The reaction was then paused and a master mix containing 4 µl of 5x RT buffer, 2 µl of 0.1 M dithiothreitol (DTT), 1 µl of 10 µM dNTPs, 0.5 µl of RNase OUT inhibitor and 0.5 µl of Superscript III (Reverse Transcriptase enzyme) was added. Samples were returned to the thermocycler and the program was continued as described the table below (table 5).

**Table 5: Reverse transcription PCR cycles**

| **Step** | **Temperature (°C)** | **Incubation time** |
|---|---|---|
| **1** | 65 | 5 min |
| **2** | 4 | 1 min |
| **3** | 25 | 10 min |
| **4** | 42 | 50 min |
| **5** | 72 | 15 min |
| **6** | 4 | 5 min |

### Quantitative PCR

Quantitative PCR (qPCR) was performed on a LightCycler 1.5 (Roche, UK) using a Quantitech SYBR green PCR kit (Qiagen, UK). 2 µl of cDNA product and 18 µl of master mix containing 10 µl SYBR green Quantitech reagent (Invitrogen, Ireland), 1 µl custom designed 25 µM primer mix and 7 µl of RNase-free dH₂O and were added to each LightCycler glass capillary. Capillaries were then closed with a small, centrifuged briefly at 2000 rpm and placed in the LightCycler 1.5. Quantitative PCR reaction was performed as outlined in table 6. For the analysis, data was normalised to the expression of β-actin and relative fold change in transcript levels were calculated using the comparative threshold method (2-ΔΔCT). Gene-specific primers were designed using Primer3 software and were purchased from Qiagen. Table 7 lists the forward and the reverse sequence for the custom-designed primers used for the experiment.

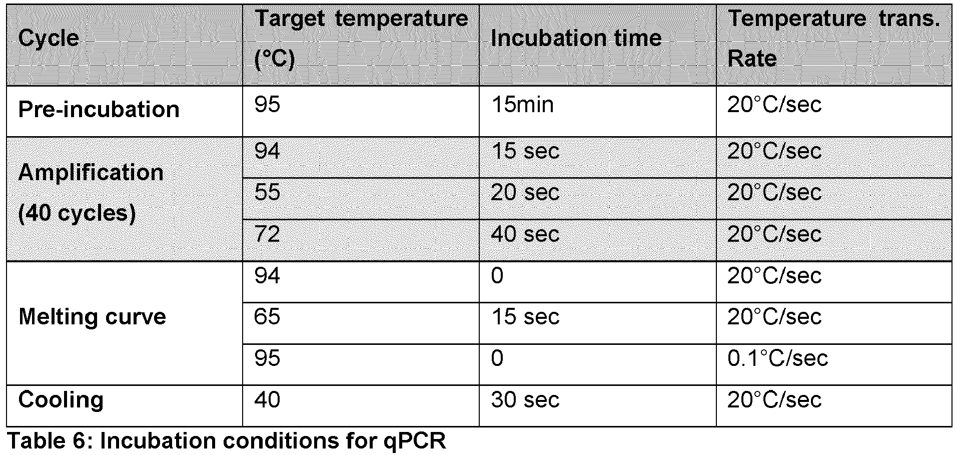

**Table 7: List of gene-specific primers**

| **Gene name** | **Forward sequence** | **Reverse sequence** |
|---|---|---|
| ***β-actin*** | 5'-AGCAGCAGACCTGACATCCT- 3' (SEQ ID NO. 7) | 5’- GTGATGCCCTTTCCAGACAT- 3’(SEQ ID NO. 8) |
| ***Scn1a*** | 5'-AGTTTGTGGACCTGGGCAAT- 3'(SEQ ID NO. 9) | 5’- ATGAAGAGCTGCAACCCGAT- 3’(SEQ ID NO. 10) |
| ***Scn2a*** | 5’- AGGAAGATGCTGTGCGGAAA- 3’(SEQ ID NO. 11) | 5'- ACCCGACCTTTGAAGCTGAA- 3'(SEQ ID NO. 12) |
| ***Scn3a*** | 5'-TCGCAGATGACAGCCACTTT- 3'(SEQ ID NO. 13) | 5’- TTCCCAGCTGCACGTAATGT- 3’(SEQ ID NO. 14) |
| ***Scn3b*** | 5'-GTTCAGCAATGCCAGCAACA- 3'(SEQ ID NO. 15) | 5’- ATTTGGAGCACAGGGGACAA- 3’(SEQ ID NO. 16) |
| ***Pcdh19*** | 5'-ATTCGCACTTCCCATCCACA- 3'(SEQ ID NO. 17) | 5’- AGTTCAGCCGCATGTTGTTG- 3’(SEQ ID NO. 18) |

### Ex vivo electrophysiology

### Slice preparation

All ex-vivo brain slices were prepared from Ant-335 or Scramble control injected mice between 2 and 4 days after surgery to ensure the maximal silencing effect of the miRNA inhibitor. For local field potential (LFP) recordings, mice were euthanised by cervical dislocation. After, brains were quickly removed from the skull, quickly dissected and submerged in oxygenated (95% O₂ and 5% CO₂) ice-cold sucrose artificial cerebrospinal fluid (ACSF; composition: 205 mM sucrose, 10 mM glucose, 26 mM NaHCO₃, 1.2 mM NaH₂PO₄.H₂O, 2.5 mM KCI, 5 mM MgCl₂, 0.1 mM CaCl₂). 400 µm thick horizontal brain slices were prepared using a vibratome (Campden 7000 smz II, Campden Instruments, Loughborough, UK), with bath temperature held at ~1°C. Slices for electrophysiology were transferred and stored at room temperature in a submerged-style holding chamber containing oxygenated recording ACSF (125 mM NaCl, 10 mM glucose, 26 mM NaHCO₃, 1.25 mM NaH₂PO₄.H₂O, 3 mM KCI, 1 mM MgCl₂ and 2 mM CaCl₂).

For patch-clamp experiments, mice were euthanised by anaesthetic overdose with sodium pentobarbital (Dolethal, Vetoqinol, UK) and transcardially perfused with ice-cold oxygenated sucrose ACSF slicing solution (205 mM sucrose, 10 mM glucose, 26 mM NaHCO₃, 1.2 mM NaH₂PO₄.H₂O, 2.5 mM KCI, 5 mM MgCl₂ and 0.1 mM CaCl₂).

### Electrophysiological recordings

Ex-vivo slices were used either for local field potential (LFP) or patch clamp recordings. All slice recordings were performed using a membrane chamber (Morris et al., 2016; Hill and Greenfield, 2011) perfused with oxygenated ACSF at a rate of 16 ml/min and heated to 34°C. Slices were allowed to equilibrate to these conditions for one hour prior to recording.

For extracellular LFP recordings, a stimulation electrode (SS3CEA4-200, MicroProbes, MD, USA) was placed in the CA3 region to stimulate the Schaffer Collateral pathway and 10 ms duration current pulses were delivered using a DS3 isolated current stimulator (Digitimer, Welwyn Garden City, UK). The response in CA1 stratum radiatum (SR) and stratum pyramidale (SP) was recorded by two extracellular borosilicate glass microelectrodes (~3 MΩ) filled with ACSF.

For patch-clamp recordings, a ~5 MΩ glass microelectrode filled with intracellular solution (135 mM K-gluconate, 4 mM KCI, 10 mM HEPES, 4 mM Mg-ATP, 0.3 mM Na-GTP, 10 mM Na₂-phosphocreatine; pH 7.3; 290 mOsm) was used. After approximately 5 minutes, a series of hyperpolarizing and depolarizing current steps (100 ms current injections with 1 second between steps, -100 to +400 pA in 25 pA increments) were injected into the neurons. The first action potential which was evoked by a depolarizing current was selected for the analysis. All recordings were performed with bridge balance compensated and access resistance < 20 MΩ. Recording was rejected if action potential did not overshoot 0 mV.

Signal was acquired using a MultiClamp 700B amplifier (Molecular Devices, CA, USA), Power 1401 digitiser (Cambridge Electronic Design (CED), Cambridge, UK) and Signal software (v6, CED). Signals were digitized at 25 kHz and low pass filtered at 10 kHz. Data were analysed using Signal v6. Population spike was measured as the maximum trough amplitude recorded from CA1 SP and population synaptic potential as the slope of the response recorded in CA1 SR.

### RESULTS

As shown in Figure 3, miR-335 levels are upregulated in two different types of epilepsy. Levels of miR-335 are upregulated in human temporal lobe epilepsy (P = 0.017) and in a mouse model of Dravet syndrome (as identified by RT-qPCR). In addition, this study showed that naive mice treated with CBD show decreased levels of miR-335 (n = 8-9 per group) compared to control group.

In silico predictions of miR-335 target genes revealed miR-335 targets many different types of channels, including sodium channels. SCN2A is amongst the highest confidence predictions (dark blue colour; Figure 4).

The results of Figure 5 show that Ant-335 upregulates the expression of different sodium channels in mouse brain. Ant-335 reduces the expression of miR-335 (P = 0.012, n = 9-11 per group). This results in the upregulation of *Scn1a* (P = 0.0009, B), *Scn2a* (P = 0.012, C) and *Scn3a* (P = 0.0287, D), where levels of *Scn3b* (E) are unchanged (n = 9-10 per group).

Figure 6 illustrates Ant-335 increases excitability of hippocampal pyramidal neurons in line with an increase in functional voltage-gated sodium channels. Ant-335 increased action potential amplitude (p=0.012) (Figure 6A). Action potential width did not change significantly as shown in Figure 6B. The rising slope (Figure 6C) and rise time (Figure 6D) were significantly accelerated by ant-335 (p=0.0016 and p=0.0043 respectively), indicative of enhanced voltage-gate sodium current. Action potential decay time (Figure 6E) and slope (Figure 6F) were not significantly changed, highlighting the specificity of the effect on voltage-gated sodium currents. Ant-335 increased the maximum firing frequency of pyramidal neurons, which were able to fire above the plateau seen in control neurons (RM two-way ANOVA current x treatment p<0.0001) as shown in Figure 6G.

Figure 7 shows that Ant-335 treatment increases susceptibility to tonic-clonic seizures in PTZ mouse model.

Figure 8 illustrates that AAV9-miR-335 upregulates the expression of miR-335 in mouse brain and Figure 9 shows that AAV9-miR-335 decreass the seizure severity in the PTZ mouse model.

The interaction between miR-335-5p and SCN2A is preserved in human brain and this is illustrated by Figure 10. iCLIP data from human epilepsy patients identified that the SCN2A gene which is located in chromosome 2, has a target site for miR-335-5p. The orange bars represent the aggregated iCLIP reads across all the patient samples that map along the SCN2A gene.

### Equivalents

The description details presently preferred embodiments of the present invention. Numerous modifications and variations in practice thereof are expected to occur to those skilled in the art

## Claims

1. A modulator of miR-335 for use in the treatment or prevention of a sodium channelopathy in a subject, wherein the modulator of miR-335 is to be delivered to the brain of the subject.

2. The modulator for use of Claim 1, wherein the sodium channelopathy is a neurological condition.

3. The modulator for use of Claim 2, wherein the neurological condition is one associated with the occurrence of seizures.

4. The modulator for use of any one of the preceding claims, wherein the sodium channelopathy is associated with, or caused by, a mutation in one or more of SCN1A, SNC2A, SNC3A, SCN4A, SCN8A, SCN9A and NALCN.

5. The modulator for use of Claim 4, wherein the mutation is a loss of function mutation, and the modulator is an inhibitor of miR-335.

6. The modulator for use of Claim 5, wherein the mutation is a loss of function mutation in SNC2A and the sodium channelopathy is an SCN2A sodium channelopathy is selected from the group comprising autism, autism spectrum disorder (ASD), and intellectual disability with later onset mild epilepsy or without epilepsy, or wherein the mutation is a loss of function mutation in SNC1A and the sodium channelopathy is an SCN1A sodium channelopathy is selected from the group comprising Dravet syndrome, developmental and epileptic encephalopathy 6, generalised epilepsy with febrile seizures plus type 2 and severe myoclonic epilepsy of infancy (SMEI).

7. The modulator for use of Claim 5, wherein the mutation is a loss of function mutation in SNC3A and the sodium channelopathy is a disease associated with p.Leu247Pro mutation in Nav1.3, or wherein the mutation is a loss of function mutation in SNC9A and the sodium channelopathy is an SCN9A sodium channelopathy is selected from the group complete insensitivity to pain (CIP), anosmia (loss of sense of smell), and primary erythromelalgia (PEM).

8. The modulator for use of Claim 4, wherein the mutation is a gain of function mutation, and the modulator is one that increases miR-355 activity.

9. The modulator for use of Claim 10, wherein the mutation is a gain of function mutation in SNC2A and the sodium channelopathy is an SCN2A sodium channelopathy selected from the group comprising epilepsy, preferably developmental and epileptic encephalopathy 11 (DEE11), episodic ataxia type 9 (EA9), benign familial infantile seizures-3 (BFIS3), and Ohtahara syndrome, or wherein the mutation is a gain of function mutation in SNC1A and the sodium channelopathy is an SCN1A sodium channelopathy selected from familial hemiplegic mirgraine-3 and a disease associated with T226M NaV.1.1 mutation.

10. The modulator for use of Claim 10, wherein the mutation is a gain of function mutation in SNC3A and the sodium channelopathy is an SCN3A sodium channelopathy selected from developmental and epileptic encephalopathy 62, familial focal epilepsy with variable foci-4 and infantile onset epileptic encephalopathy, or wherein the mutation is a gain of function mutation in SNC9A and the sodium channelopathy is an SCN9A sodium channelopathy selected from paroxysmal extreme pain disorder (PEPD) and inherited erythromelalgia (IEM).

11. A modulator of miR-335 for use in the treatment of a UBE3A associated condition in a subject.

12. The modulator for use of Claim 11, wherein condition is Angelman's syndrome or Prader-Willi Syndrome.

13. The modulator for use of any one of Claims 1 to 7 and 11 to 12, wherein the modulator is an inhibitor of miR-335.

14. The modulator for use of Claim 13, wherein the inhibitor is selected from the group comprising: antagomirs, anti-MIRs, microRNA sponges, tiny seed-targeting LNA oligonucleotides, antisense oligonucleotides, short-interfering RNA, decoy oligonucleotides, aptamers, and antibodies that specifically recognize DNA:RNA heteroduplexes.

15. The modulator of any one of Claims 1 to 4 and 8 to 10, wherein the modulator is one that increases miR-335, preferably, wherein the modulator is miR-335 or a miR-335 mimic.
